# EUROPEAN PATENT SPECIFICATION

(11) **EP 1 680 128 B1**
(45) Date of publication and mention of the grant of the patent: **14.02.2007**
(21) Application number: 04798764.9
(22) Date of filing: 03.11.2004
(51) Int. Cl.: A61K 31/6615, A61P 43/00, A61P 3/14, A61K 9/70

(54) **MYO-INOSITOL HEXAPHOSPHATE FOR TOPICAL USE**
MYO-INOSITOL HEXAPHOSPHAT ZUR TOPISCHEN VERWENDUNG
HEXAPHOSPHATE DE MYO-INOSITOL POUR USAGE TOPIQUE

(30) Priority: 07.11.2003 ES 200302600
(43) Date of publication of application: 19.07.2006
(73) Proprietor: Laboratorios Sanifit, S.L., 07350 Binissalem (ES)
(72) Inventor: GRASES FREIXEDAS, F., Uni. de Les Illes Balears, 07071 Palma de Mallorca (ES); PERELLO BESTARD, J., Uni. de Les Illes Balears, 07071 Palma de Mallorca (ES); ISERN AMENGUAL, B., Uni. de Les Illes Balears, 07071 Palma de Mallorca (ES); PRIETO ALMIRALL, R., Uni. de Les Illes Balears, 07071 Palma de Mallorca (ES); COSTA BAUZÀ , A., Uni. de Les Illes Balears, 07071 Palma de Mallorca (ES)
(74) Representative: Ponti Sales, Adelaida
(86) International application number: PCT/IB2004/003588
(87) International publication number: WO 2005/044278

(56) References cited:
- US-A- 5 082 833
- US-A- 5 268 176
- VAN DEN BERG, C. J. ET AL: "Inositol phosphates and phytic acid as inhibitors of biological calcification in the rat" CLINICAL SCIENCE , 43(3), 377-83 CODEN: CSCIAE; ISSN: 0143-5221, 1972, XP008042000
- GRASES, F. ET AL: "Phytate prevents tissue calcifications in female rats" BIOFACTORS , 11(3), 171-177 CODEN: BIFAEU; ISSN: 0951-6433, 2000, XP008041991
- GRASES, F. ET AL: "Effects of phytic acid on renal stone formation in rats" SCANDINAVIAN JOURNAL OF UROLOGY AND NEPHROLOGY , 32(4), 261-265 CODEN: SJUNAS; ISSN: 0036-5599, 1998, XP008041988
- KASTING G B ET AL: "SKIN PENETRATION ENHANCEMENT METHODS FOR HYDROPHILIC COMPOUNDS" PHARMACEUTICAL RESEARCH, NEW YORK, NY, US, vol. 14, no. 11, SUPPL, November 1997 (1997-11), page S452, XP001105174 ISSN: 0724-8741
- GRASES, F. ET AL: "Dietary myo-inositol hexaphosphate prevents dystrophic calcifications in soft tissues: a pilot study in Wistar rats" LIFE SCIENCES , 75(1), 11-19 CODEN: LIFSAK; ISSN: 0024-3205, 2004, XP008041984
- GRASES ET AL UROLOGICAL RESEARCH vol. 22, no. 1, 1994, pages 39 - 43
- MANARY ET AL AMERICAN SOCIETY FOR NUTRITIONAL SCIENCES 2000, pages 2959 - 2964

## Description

### TECHNICAL FIELD

The present invention relates to the field of products with dermatological and systemic activity.

In particular, the present invention relates to a composition which includes myo-inositol hexaphosphate applied by topical administration for use in the treatment of a disease associated with the formation of heterogeneous nucleants inducing the development of pathological calcifications and its use for the manufacture of a medicament for the treatment and/or prevention of pathological calcifications.

### STATE OF THE ART

Ectopic calcifications are common alterations associated with soft tissues, mainly skin, kidney, tendons and cardiovascular tissues.

All the extracellular fluids in mammals are supersaturated in relation to calcium phosphate (hydroxiapatite) and are therefore metastable in respect of this solid. However, these crystals do not precipitate spontaneously. Physiologically, crystallisations only take place in controlled situations such as in the formation of teeth or bone.

Uncontrolled pathological crystallisations are nevertheless also frequent. Indeed, crystallisation does not take place indiscriminately in all biological fluids, since it depends not only on thermodynamic factors (supersaturation) but also on kinetic factors. Thus, biological calcifications dependents mainly on three factors: supersaturation (thermodynamic factor), the presence of heterogeneous nucleants, and/or the presence of crystallisation inhibitors (kinetic factors). It is now known that the presence of damaged tissue provides heterogeneous nucleants that serve as substrates for the initial formation of crystals (Valente M, Bortolotti U & Thiene G. (1985) Ultrastructural substrates of dystrophic calcification in porcine bioprosthetic valve failure. *American Journal of Pathology* 119, 12-21).

On the other hand, the action of the so-called crystallisation inhibitors can slow down or prevent the formation of crystals, although these processes are rather little known. When the inhibition mechanisms disappear the calcium crystals precipitate and proliferate.

Myo-inositol hexaphosphate (Ins*P*₆, phytate) is an important component of plant seeds which has been shown to have potent capacity as an inhibitor of the crystallisation of calcium salts in urine (Grases F, Garcia-Ferragut L, Costa-Bauza A & March JG (1996) Study of the effects of different substances on the early stages of papillary stone formation. *Nephron* **73**, 561-568; Grases F, Garcia-Ferragut L & Costa-Bauza A (1998a) Development of calcium oxalate crystals on urothelium: effect of free radicals. *Nephron* **78**, 296-301; Grases F, Garcia-Gonzalez R, Torres JJ & Llobera A (1998b) Effects of phytic acid on renal stone formation in rats. *Scandinavian Journal of Urology and Nephrology* **32**, 261-265). Phytate has also been shown to prevent tissue calcification in rats (clinical science (1972) 43,377-383, Biofactors 11 (2000), 171-177. All grain cereals (such as maize, wheat and rice) contain around 1%, while other foods such as soya, peanuts or sesame contain 1.5% or more. In most seeds the phytate is associated with calcium and magnesium ions (forming the salt known as phytine) and is not distributed homogeneously in the seed. For example, the endosperm of wheat and rice grains contains practically no phytate, since it is concentrated in the germ and in the aleuronic layers of the grain cells and in the bark. Maize differs from most cereals in that nearly 90% of the phytate is concentrated in the germ of the grain, as occurs with carob germ.

It has also been shown that the levels of phytate in the blood and tissues of mammals clearly depends on its ingestion through the diet (Grases F, Simonet BM, Prieto RM & March JG (2001a) Phytate levels in diverse rat tissues: influence of dietary phytate. *British Journal of Nutrition* **86**, 225-231; Grases F, Simonet BM, Prieto RM & March JG (2001b) Variation of *Ins*P₄, *Ins*P₅ and *Ins*P₆ levels in tissues and biological fluids depending on dietary phytate. *The Journal of Nutritional Biochemistry* **12**, 595-601).

### OBJECT OF THE INVENTION

The object of this invention is to find new applications of myo-inositol hexaphosphate (hereinafter referred to as "phytate") related with the properties described in the state of the art.

The object of this invention is a composition including phytate applied by topical administration for use in the treatment of diseases associated with the formation of heterogeneous nucleants that induce the development of pathological calcifications, both subepithelial and in other soft tissues of the organism.

The applications for phytate disclosed below have not been described before and their use can be beneficial in the treatment of certain diseases. In particular, it has been found that the composition including phytate applied by topical administration has an activity that inhibits the growth of heterogeneous nucleants and the formation of crystals of calcium salts.

In this invention, the new applications of phytate are explained using experimental models. These analysis models indicate that a composition including phytate applied by topical administration can be used for the manufacture of a medicament for the treatment of diseases in soft tissues due to its effect as an inhibiting agent against the development of heterogeneous nucleants of crystallisation of calcium salts.

### DESCRIPTION OF THE INVENTION

In the present invention, "phytate" or "myo-inositol hexaphosphate" are taken to mean the molecule corresponding to the formula: 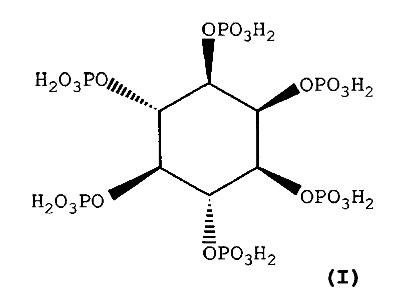 and pharmaceutically acceptable salts thereof, which include sodium, potassium, calcium, magnesium or calcium-magnesium salts.

In the present invention, "crystallisation nucleant" is taken to mean a substance that serves as a substrate for the initial formation of crystals, acting as an inducer of the development of pathological calcifications, both subepithelial and in other soft tissues of the organism.

The object of this invention is a composition including myo-inositol phosphate (hereinafter referred to as "phytate") applied by topical administration for use in the treatment of diseases associated with the formation of heterogeneous nucleants in a soft tissue.

It is well-known by those skilled in the art that the skin constitutes one of human beings' main protective barriers, acting, among others, as a barrier against microorganisms and chemical substances; as a barrier to certain forms of energy (heat, light, etc). The stratum corneum constitutes the real barrier against xenobiotics in general, and drugs in particular, passing through the skin. The protective action of the stratum corneum is due to its inherent structure, in which the main component (by weight) is keratin, together with variable proportions of intrinsic lipids coming from cutaneous surface secretion.

Also known is the fact that a drug has to reach the site of action in order to give rise to a pharmacological effect. When a drug is administered orally (as in the case of phytate), a great part of the active substance is metabolised in the stomach and/or liver and ceases to be active; in other words, it is a drug with low bioavailability.

Surprisingly, the inventors of this invention have found that phytate, with a high negative charge, can be absorbed by the skin when it is administered topically, passing into the bloodstream and acting on the damaged zone (in which a heterogeneous nucleant would have been generated).

Therefore, with a composition in accordance with the object of the present invention the bioavailability of the phytate is improved, because when it is applied onto the skin, it is absorbed and exercises a local and systemic effect, thereby avoiding the metabolisation that it can undergo in oral administration.

In one embodiment of this invention, said composition, including phytate applied by topical administration, can be used for the treatment of a disease which consists on the formation of calcifications in a soft tissue.

In another embodiment, said soft tissue is a subepithelial tissue, a blood vessel wall, or a renal, pulmonary or cerebral tissue.

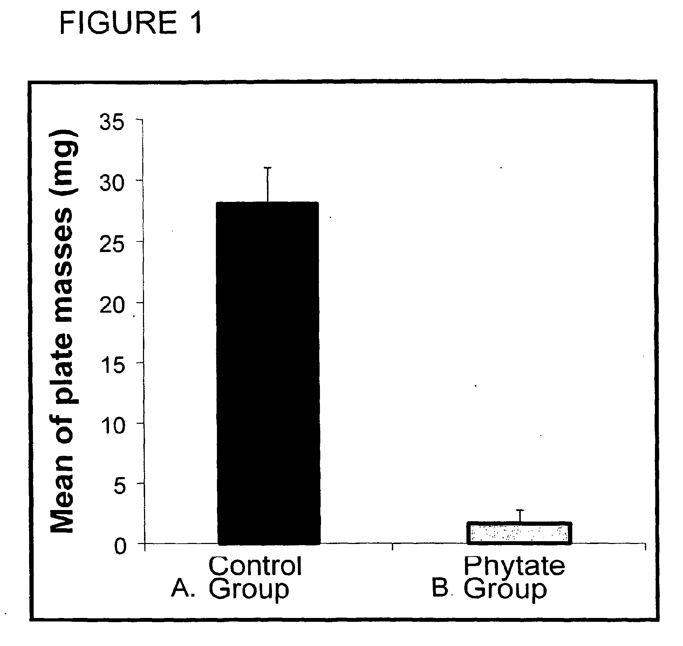

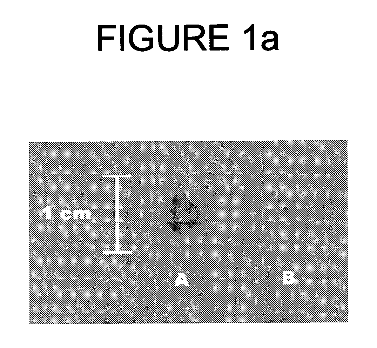

In *in vivo* models it has been found, for example, that with a composition which includes 2% of phytate (w/w) together with excipients such as those described in Example 2, the size of the calcification plates diminishes, and this is accompanied by a significant increase in the concentrations of plasmatic and urinary phytate (showing that the phytate is absorbed by the skin), as shown in Figure 1.

These analysis models therefore indicate that a composition including phytate applied by topical administration can be used for the manufacture of a medicament for the treatment of a disease associated with the formation of heterogeneous nucleants, preferably of a disease associated with the formation of calcifications, in a soft tissue.

The compositions applied by topical administration according to the object of the present invention will include a pharmaceutically acceptable vehicle or diluent that does not reduce the therapeutic effect of the phytate and does not interfere with its absorption through the skin. Examples of pharmaceutically acceptable vehicles or diluents include, gels, creams, lotions, solutions and suspensions.

Preferably, said disease consists on a subepithelial dystrophic calcification, or an arterial, tendon or renal calcification.

### DESCRIPTION OF THE FIGURES

Figure 1 shows the effect of the phytate administered topically in the treatment and/or prevention of hydroxiapatite plates generated in Wistar rats by injection of 200 µl of 0.1% potassium permanganate subcutaneously on each of the sides of the interscapular region. Experimental conditions. Group A: diet 4068.02 (lacking in phytate) and application of 1 g of moisturising cream without phytate twice a day. Group B: diet 4068.02 and application of 1 g of moisturising cream with 2% phytate twice a day (duration of the experiment: 30 days). The image in the figure pertains to the hydroxiapatite plates extracted from group A and B rats: As can be observed, the size of the hydroxiapatite plates of the group B rats (treated with a composition according to the present invention) is significantly smaller than that of the plates extracted from group A rats (Control).

### EXAMPLES OF EMBODIMENT OF THE INVENTION

This invention is additionally illustrated by means of the following examples of the scope thereof.

### Example 1

### Formulation 1

| | |
|---|---|
| pH | 4.5 |
| Sodium phytate | 2.9% (2% phytate) |
| Almond oil | 4% |
| Isopropyl myristate | 3.8% |
| Stearic acid | 1% |
| Lactic acid | 1.6% |
| Ethyl linoleate | 2.5% |
| Glyceril stearate | 4% |
| Propyl paraben | 0.1% |
| Cetearil alcohol | 4% |
| Controx VP (lecithin, tocopherol, ascorbitol palmitate, hydrogenated citrate of palm glycerides) | 0.025% |
| Water | 70.2% |
| T.E.A. | 0.1% |
| Allantoin | 0.1% |
| Glycerine | 4.875% |
| Methyl paraben | 0.2% |
| Imidazolidinyl urea | 0.3% |
| Essence | 0.3% |

### Formulation 2

| | |
|---|---|
| pH | 4.8 |
| Sodium phytate | 0.7% (0.5% phytate) |
| Almond oil | 4% |
| Isopropyl myristate | 3.8% |
| Stearic acid | 1% |
| Lactic acid | 1.2% |
| Ethyl linoleate | 3.5% |
| Glyceril stearate | 3% |
| Propyl paraben | 0.1% |
| Cetearil alcohol | 3% |
| Controx VP (lecithin, tocopherol, ascorbitol palmitate, hydrogenated citrate of palm glycerides) | 0.025% |
| Water | 73.8% |
| T.E.A. | 0.1% |
| Allantoin | 0.1% |
| Glycerine | 4.875% |
| Methyl paraben | 0.2% |
| Imidazolidinyl urea | 0.3% |
| *Aloe barbadensis* | 0.3% |

### Formulation 3

| | |
|---|---|
| pH | 4 |
| Sodium phytate | 2.5% (1.7% phytate) |
| Almond oil | 4.5% |
| Isopropyl myristate | 3.3% |
| Stearic acid | 1.5% |
| Lactic acid | 2% |
| Ethyl linoleate | 2% |
| Glyceril stearate | 4.5% |
| Propyl paraben | 0.1% |
| Cetearil alcohol | 3% |
| Controx VP (lecithin, tocopherol, ascorbitol palmitate, hydrogenated citrate of palm glycerides) | 0.025% |
| Water | 70.72% |
| T.E.A. | 0.1% |
| Allantoin | 0.1% |
| Glycerine | 4.875% |
| Methyl paraben | 0.2% |
| Imidazolidinyl urea | 0.3% |
| Essence | 0.3% |

### Example 2:

14 male Wistar rats weighing 275-300 g (from Harlan Iberica s.l., Barcelona, Spain) were acclimatised for 7 days in our animals facility, whose temperature and humidity conditions were 21 ± 1 °C and 60 ± 5% respectively, and with light-darkness cycles of 12:12 hours. The rats were housed in Plexiglas cages, with two animals per cage, and were lived on meals and drink *ad libitum.*

Following the acclimatisation period, the animals were divided randomly into two groups, one of 8 (control group) and 6 (treated group) rats, respectively, and both groups were supplied diet 4068.02 (HopeFarms BV, Woerden, The Netherlands), a purified synthetic diet entirely lacking in phytate. Moreover, each rat of the control group had 1 g of a standard base cream (including no phytate) applied twice a day, while the treated group had the same amount of cream applied with the same frequency but with a phytate supplement, in the form of sodium salt, at 2% (corresponding to formulation no. 1). The pH of both creams was 4-4.5. This treatment was continued for 21 days.

At the end of this period, the formation of hydroxiapatite (calcium phosphate) plates was induced by subcutaneous injection of 200 µl of KMnO₄ (potassium permanganate) at 0.1% into one of the sides of the interscapular region.

KMnO₄ is a powerful antioxidant and causes local cellular necrosis at the site into which it is injected, thus leaving organic material which can act as a heterogeneous nucleant for the development of hydroxiapatite plates. These plates were left to grow for a period of 10 days and left inserted under the subcutaneous tissue layer, possibly invading part of the dermis, and were clearly visible for excision once the study had been concluded.

Finally, the animals were anaesthetised with pentobarbital (50 mg kg⁻¹, i.p.) and the plates were removed, dried and weighed.

The results obtained, shown in Figures 1 and 1a, show that the rats submitted to a phytate-poor diet generate large subepithelial plates of hydroxiapatite, while if the rats were submitted to daily application of a moisturising cream with phytate (2%), the development of the corresponding calcified plates was significantly reduced.

The procedures used in this experiment were carried out in accordance with Directive 86/609/EEC relating to the protection of animals used for experimental and scientific purposes, and official permission was requested from the ethics committee of Illes Balears University to carry out the experiment.

## Claims

1. Use of a composition including myo-inositol hexaphosphate corresponding to the formula: 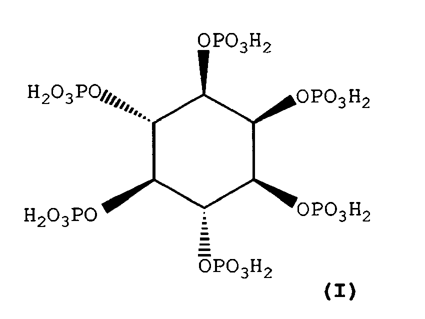 or pharmaceutically acceptable salts thereof for the manufacture of a medicament for the prevention and/or treatment by topical administration of a disease associated with the development of heterogeneous nucleants which consists on a pathological calcification in a soft tissue.

2. Use according to claim 1, in which said disease consists on a subepithelial dystrophic calcification.

3. Use according to claim 1, in which said disease consists on an arterial calcification.

4. Use according to claim 1, in which said disease consists on blood vessels calcification.

5. Use according to claim 1, in which said disease consists on a renal calcification.

6. Use according to claim 1, in which said disease consists on a cerebral calcification.

7. Use according to claim 1, in which said disease consists on a pulmonary calcification.

## Patentansprüche

1. Verwendung einer Zusammensetzung einschließlich Myo-Inositol-Hexaphosphat gemäß der Formel: 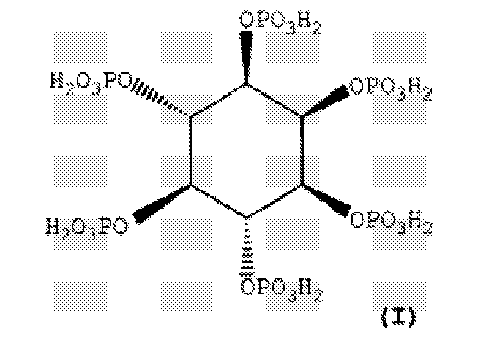 oder dessen pharmazeutisch annehmbare Salze zur Herstellung eines Medikaments für die Vorsorge und/oder Behandlung einer Krankheit, die mit der Entwicklung heterogener Keimbildner assoziiert ist, die aus einer pathologischen Kalzifikation in einem weichen Gewebe besteht, durch topische Verabreichung.

2. Verwendung gemäß Anspruch 1, wobei die Krankheit aus einer subepithelialen, dystrophen Kalzifikation besteht.

3. Verwendung gemäß Anspruch 1, wobei die Krankheit aus einer arteriellen Kalzifikation besteht.

4. Verwendung gemäß Anspruch 1, wobei die Krankheit aus einer Blutgefäß-Kalzifikation besteht.

5. Verwendung gemäß Anspruch 1, wobei die Krankheit aus einer renalen Kalzifikation besteht.

6. Verwendung gemäß Anspruch 1, wobei die Krankheit aus einer zerebralen Kalzifikation besteht.

7. Verwendung gemäß Anspruch 1, wobei die Krankheit aus einer pulmonaren Kalzifikation besteht.

## Revendications

1. Utilisation d'une composition comprenant de l'hexaphosphate de méso-inositol correspondant à la formule : 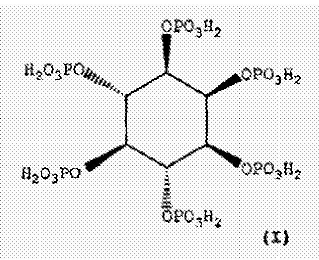 ou des sels pharmaceutiquement acceptables de celui-ci pour la fabrication d'un médicament destiné à la prévention et/ou au traitement, par administration topique, d'une maladie associée au développement de nucléants hétérogènes qui consiste en une calcification pathologique dans un tissu mou.

2. Utilisation selon la revendication 1, dans laquelle ladite maladie consiste en une calcification dystrophique sous-épithéliale.

3. Utilisation selon la revendication 1, dans laquelle ladite maladie consiste en une calcification artérielle.

4. Utilisation selon la revendication 1, dans laquelle ladite maladie consiste en une calcification des vaisseaux sanguins.

5. Utilisation selon la revendication 1, dans laquelle ladite maladie consiste en une calcification rénale.

6. Utilisation selon la revendication 1, dans laquelle ladite maladie consiste en une calcification cérébrale.

7. Utilisation selon la revendication 1, dans laquelle ladite maladie consiste en une calcification pulmonaire.
